# EUROPEAN PATENT APPLICATION

(11) **EP 2 604 687 A1**
(43) Date of publication of application: **19.06.2013**
(21) Application number: 12192631.5
(22) Date of filing: 14.11.2012
(51) Int. Cl.: C12N 11/08, B09B 3/00

(54) **Carrier for enzyme immobilization, method of preparation thereof, method of enzyme immobilization, and biocatalyst**

(30) Priority: 12.12.2011 CZ 20110811; 12.12.2011 CZ 201125303 U
(71) Applicant: Vysoké Ucení Technické V Brne, 601 90 Brno (CZ)
(72) Inventor: Omelkova, Jirina, 613 00 Brno (CZ); Zichova, Miroslava, 152 00 Praha 52 - Hlubocepy (CZ)
(74) Representative: Hartvichova, Katerina

(57) **Abstract**

The present invention provides a carrier for enzyme immobilization which comprises crushed PET packaging material having the particle size of less than 0.8 mm, activated by acetone. This carrier is suitable in particular for the immobilization of glycanol hydrolases. The invention includes also a method for preparation of the carrier, a method for enzyme immobilization, and the resulting biocatalyst.

## Description

### Field of Art

The present invention relates to the field of recycling of PET packaging materials and to the field of enzyme immobilization.

### Background Art

Insoluble forms of enzymes, i.e., enzymes anchored on an insoluble carrier, are very important in modern technological processes. The immobilized enzymes can be utilized in biotechnological processes in food-processing and pharmaceutical industries, for recyclation of wastes into various bioproducts, etc.

Various carriers are known for enzyme immobilization, but their price substantially increases the costs of preparation of insoluble enzyme forms. For the immobilization of enzymes which catalyze polymeric substrate degradation, the common immobilization processes using entrapment, encapsulation, or cross-linking are not suitable. Thereby, the choice of the commercially available carriers is limited. The carries allowing the immobilization by covalent binding are often unsuitable for food-processing industry applications, in particular when the enzyme-carrier bond is formed by chemicals which are not allowed by norms and standards for food-processing (Roger A. Sheldon ,Enzyme Immobilization: The Quest for Optimum Performance, Special Issue: Biocatalysis Volume 349, Issue 8-9, pages 1289-1307, June 4, 2007 Wiley Online; R.A.Sheldon Enzyme immobilization: The quest for optimum performance., Adv.Synth.Catal. 349 (2007) 1289-1307).

There is a need for a suitable enzyme immobilization carrier which would be cost-effective, ecological and applicable in food-processing industry.

### Disclosure of the Invention

The object of the invention is a carrier for enzyme immobilization, which contains crushed PET packaging materials (PET = poly(ethylene terephthalate)) having the particle size of less than 0.8 mm, activated by acetone.

PET packaging materials include for example PET bottles. This method is thus suitable, e.g., for recycling waste PET bottles, in particular colourless PET bottles.

The carrier of the invention is suitable for immobilization of enzymes, in particular glycanohydrolases.

A further object of the invention is a method of preparation of the carrier for enzyme immobilization, which comprises the steps of crushing PET packaging materials, isolating the fraction having the particle size of less than 0.8 mm, activating said fraction by treatment in acetone for a period of 5 to 10 hours at stirring, and drying said activated fraction.

Preferably, the activation by acetone is performed at room temperature.

Preferably, the PET packaging materials are crushed on a cutting mill and the fractions are obtained by means of sieves.

The enzyme immobilization is carried out by means of adsorption of an enzyme on the carrier, at room temperature, in a buffer at the pH which is optimum for the enzyme activity. This does not require any further cost- or time-demanding steps.

The scope of the invention further includes a biocatalyst which contains an enzyme adsorbed on the carrier of the present invention. Preferably, the enzyme is glycano hydrolase.

So far, no preparation of a carrier suitable for immobilization of any biologically active compound from the PET packaging materials was reported. However, PET packaging materials are commonly used in the food-processing industry, so when using enzymes immobilized on a carrier according to the present invention, the foodstuffs are not exposed to substances (carriers) the use of which in the food-processing industry would be forbidden or unsuitable.

The carrier produced from the waste material by the process of the present invention has excellent sorption and mechanical properties for use for enzyme immobilization and for a subsequent application in technological processes. The application of the thus produced biocatalyst (i.e., the carrier with the immobilized enzyme) allows an easier regulation of the enzyme catalytic activity, the enzyme can be removed from a process and can be repeatedly used in another technological process. The biocatalyst of the invention also has a higher thermal stability than free enzyme.

### Examples of Carrying Out the Invention

### Example 1: Preparation of the carrier

160 g of PET packaging materials were cut into squares ca 3 cm x 3 cm, which were further crushed using a cutting mill. The crushed material was fractioned using a set of sieves. The fraction having the particle size of 0.8 to 0.4 mm was used for the activation: 60 g of this fraction was weighed and mixed with 250 ml of acetone, and activated at room temperature using a laboratory shaker for a period of 5 to 10 hours. After the activation is completed, the carrier is dryed by drawing off acetone.

### Example 2: Immobilization of an enzyme

1 g of the activated carrier prepared in Example 1 is suspended in 20 ml of 0.1M acetate buffer having the pH 4.8, and the surface of the carrier is wetted by intensive stirring for 1 hours at room temperature. 5 µl of commercial cellulase in 1 ml acetate buffer is added to the suspension under continued stirring. After 5-hour stirring at room temperature, the immobilized system is recovered by filtration using a fritted funnel.

### Example 3: Use of the immobilized enzyme

The activity of the immobilized enzyme was verified by batch application. The reaction mixture was composed of 1 g of the immobilized system (biocatalyst), produced in example 2, in 5 ml of 0.1M acetate buffer (pH 4.8) and 5 ml of 1% carboxy methyl cellulose solution in 0.1M acetate buffer (pH 4.8). The activity of the immobilized enzyme was monitored using the increase of the amount of reducing saccharides in the reaction mixture. The relative activity of the immobilized system was 36 %.

## Claims

1. A carrier for enzyme immobilization, **characterized in that** it contains crushed PET packaging material having the particle size of less than 0.8 mm, activated by acetone.

2. A method of preparation of the carrier for enzyme immobilization of claim 1, **characterized in that** it comprises the steps of crushing PET packaging material, isolating the fraction having the particle size of less than 0.8 mm, activating said fraction by treatment in acetone for a period of 5 to 10 hours at stirring, and drying said activated fraction.

3. The method according to claim 2, wherein the activation by acetone is performed at room temperature.

4. The method according to claim 2 or 3, wherein the activation by acetone is perfomed for the period of 5 hours.

5. A method of immobilization of an enzyme on the carrier of claim 1, **characterized in that** the enzyme is adsorbed to the carrier at room temperature in a buffer at the pH which is optimum for the enzyme activity.

6. A biocatalyst, **characterized in that** it contains an enzyme adsorbed on the carrier according to claim 1.

7. The biocatalyst according to claim 6, wherein the enzyme is glycano hydrolase.
